# EUROPEAN PATENT APPLICATION

(11) **EP 1 821 054 A2**
(43) Date of publication of application: **22.08.2007**
(21) Application number: 06122676.7
(22) Date of filing: 20.10.2006
(51) Int. Cl.: F26B 11/14, F26B 23/02

(54) **Food waste drying apparatus**

(30) Priority: 15.02.2006 KR 20060014611
(71) Applicant: Loofenlee Co., Ltd., Seoul (KR)
(72) Inventor: Lee, Hee Ja, 110, Hyundai Villa 61-2, Seoul 135-090 (KR)
(74) Representative: Szynka, Dirk

(57) **Abstract**

The food waste drying apparatus of the present invention having a main body (10), a food waste receiving tub (16) provided in the main body, a heater (24) heating and drying food waste contained in the food waste receiving tub (16), and a suction fan (32) and a circulation fan (22) circulating some heated moist air and simultaneously discharge some moist air to the outside, the apparatus comprising: a rotating shaft (52) provided on the support (14), and an agitation means (58) provided on the food waste receiving tub (16) and coupled to the rotating shaft (52). To reduce a temperature difference in the food waste, the food waste in the food waste receiving tub (16) is agitated by the agitation means (58). Also, to burn odorous particles in the discharged moist air a deodorizing unit is provided.

## Description

### BACKGROUND OF THE INVENTION

### 1. Field of the Invention

The present invention relates generally to food waste drying apparatuses and, more particularly, to a food waste drying apparatus which reduces heating temperature differences induced in food waste during a drying process, thus maximizing the drying efficiency, and which heats odorous particles, generated during a process of heating and drying food waste, to a high temperature, thus completely burning the odorous particles before discharging them.

### 2. Description of the Related Art

Recently, because Ban on directly disposing food waste into landfills has been enforced in order to solve the problem of environmental pollution due to food waste, various kinds of apparatuses for food waste treatment have been proposed. A food waste crushing and drying apparatus, which dries food waste after finely crushing the food waste using a crusher to reduce the volume thereof, and a food waste drying apparatus, which dries food waste to reduce the volume thereof, are representative examples of the apparatuses for food waste treatment. However, because the food waste crushing and drying apparatus generates noise and odors while crushing food waste, the food waste drying apparatus that evaporates food waste before discharging it has been mainly used.

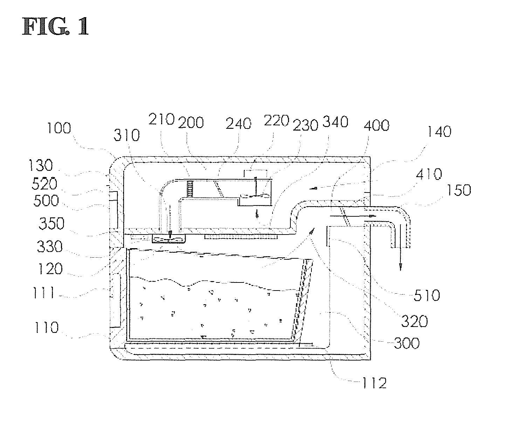

A representative example of such conventional food waste drying apparatuses is shown in FIG. 1. As shown in FIG. 1, the conventional food waste drying apparatus includes a housing 100, the interior space of which is partitioned by a partition wall 160 into upper and lower spaces. A separate receiving space 300 is defined in the lower space of the housing 100. A slide rail 112 is provided on the bottom of the receiving space 300. A drawer type support casing 110, on which a food waste receiving tub 120 containing food waste therein is seated, is slidably coupled to the slide rail 112 so as to be movable forwards and backwards. An air outlet duct 150 is provided behind the drawer type support casing 110, so that moisture and odors, which are generated from the food waste by a heating and drying means that will be described later herein, are discharged outside through the air outlet duct 150.

In the upper space of the housing 100 defined by the partition wall 160, a suction fan 230, which draws air in through an air inlet 140, a first backflow prevention member 240, which prevents odors of food waste from flowing backwards, a heater 210, which heats drawn air, and a hot air discharge fan 330, which supplies air heated by the heater 210 into the food waste receiving tub 120 of the drawer type support casing 110, are consecutively installed in an air suction pipe 200.

Furthermore, a far infrared film heater 340, which reheats food waste and radiates far infrared rays to sterilize bacilli and microbes, is provided under the lower surface of the partition wall 160. A second backflow prevention member 410 is provided in the air outlet duct 150 to prevent air from flowing backwards from the outside.

However, the conventional food waste drying apparatus having the above-mentioned construction has the following problems.

First, in the food waste receiving tub, there is an excessive temperature difference between an upper portion of food waste which is disposed adjacent to the hot air discharge fan and a lower portion of food waste which is spaced apart from the hot air discharge fan by a relatively long distance. Therefore, a long time is required to completely dry the food waste. Thereby, the electric heating cost is increased and, as well, the drying operation is not perfectly conducted.

Second, the conventional food waste drying apparatus has a structure such that odors generated during the drying process are directly discharged outside the apparatus, thus being injurious to neighbors.

### SUMMARY OF THE INVENTION

Accordingly, the present invention has been made keeping in mind the above problems occurring in the prior art, and an object of the present invention is to provide a food waste drying apparatus which reduces heating temperature differences induced in food waste during a drying process, thus maximizing the drying efficiency, and which heats odorous particles, generated during a process of heating and drying food waste, to a high temperature, thus completely burning the odorous particles before discharging them.

In order to accomplish the above object, the present invention provides a food waste drying apparatus, comprising: a main body; a food waste receiving tub seated on a support provided in the main body; a heater to heat and dry food waste contained in the food waste receiving tub; and a suction fan and a suction pipe to discharge heated moist air therethrough to an outside. The food waste drying apparatus further comprises: a rotating shaft that is vertically provided below the support and rotates using a driving force transmitted from a drive motor; agitation means provided on a bottom of the food waste receiving tub and removably coupled to the rotating shaft, so that the agitation means agitates the food waste, thus reducing a temperature difference in the food waste, thereby increasing a drying efficiency; and a deodorizing unit disposed between the food waste receiving tub and the support and provided in a path of the suction pipe to burn the moist air, which contains odorous particles therein and is discharged through the suction pipe.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a sectional view showing the construction of a conventional food waste drying apparatus;
FIG. 2 is a sectional view of a food waste drying apparatus, according to an embodiment of the present invention; and
FIG. 3 is an enlarged sectional view of a deodorizing unit of the food waste drying apparatus of FIG. 2.

### DESCRIPTION OF THE PREFERRED EMBODIMENTS

Hereinafter, an embodiment of the present invention will be described in detail with reference to the attached drawings.

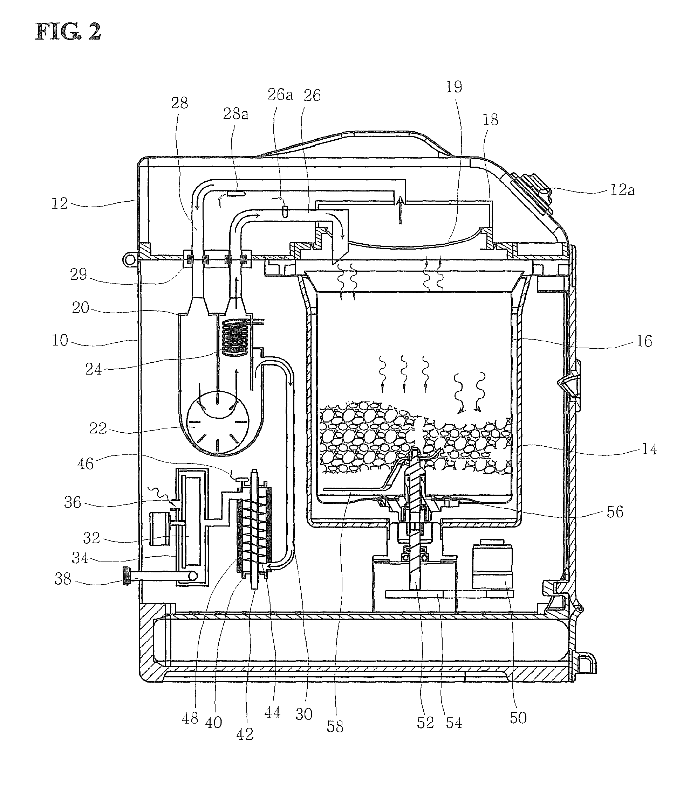

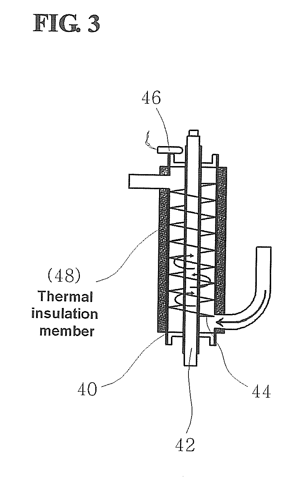

FIG. 2 is a sectional view of a food waste drying apparatus, according to the embodiment of the present invention. FIG. 3 is an enlarged sectional view of a deodorizing unit shown in FIG. 2.

As shown in FIG. 2, the food waste drying apparatus of the present invention includes a main body 10, which forms an outer casing of the apparatus, and an upper lid 12, which is hinged to the main body 10 so as to be openable and has a control panel 12a thereon to allow a user to control the apparatus.

Furthermore, a tub support 14, which has a predetermined inner diameter and supports a food waste receiving tub 16, is installed at a lower position in the main body 10. The food waste receiving tub 16 has an outer diameter less than the inner diameter of the tub support 14 such that it is seated into the tub support 14.

As well, a sealing member 18 is provided in the upper lid 12, so that, when the upper lid 12 is closed, an airtight drying space is defined by the food waste receiving tub 16 together with the sealing member 18.

The food waste drying apparatus of the present invention further includes an air circulation device 20, which supplies hot air to food waste, contained in the food waste receiving tub 16, to dry it. The air circulation device 20 occupies a space from the inside of the upper lid 12, which faces the food waste receiving tub 16, to a position around the sidewall of the tub support 14. The air circulation device 20 includes a circulation fan 22, which is operated by driving force of a motor and circulates air in the air circulation device 20, and a main heater 24, which is installed at a predetermined position in the air circulation device 20. The air circulation device 20 further includes an air supply pipe 26, which has a discharge port and supplies hot air, generated by the circulation fan 22 and the main heater 24, into the food waste receiving tub 16, and an induction pipe 28, through which moist air, which is generated by the hot air supplied from the air supply pipe 26, is drawn into the air circulation device 20 to recirculate the air.

In the present invention, a temperature sensor 26a is provided on the air supply pipe 26 to detect the temperature of the main heater 24, thus preventing the apparatus from being damaged by overheating. A moist air sensor 28a, which serves as a means for automatically turning off the apparatus depending on the state of moist air that recirculates, is provided on the induction pipe 28.

Meanwhile, the possible arrangement locations of the air supply pipe 26 and the induction pipe 28 of the air circulation device 20 must include the inside spaces of the upper lid 12 and the main body 10. Therefore, each pipe has a separable structure. A pipe connection box 29 is installed at a boundary between the upper lid 12 and the main body 10, such that, when the upper lid 12 is closed, each pipe enters the state of being a single body.

Furthermore, a suction pipe 30 is connected to a predetermined portion of the air circulation device 20, thus bypassing some moist air which recirculates. A suction fan 32, which is operated by a motor and forcibly draws and discharges some moist air outside the apparatus, is provided in a fan box 34 coupled to a rear end of the suction pipe 30. An air inlet 36, which makes it possible to smoothly cool heated air using outside air drawn therethrough, and an air outlet 38, which discharge the drawn air outside the apparatus, are formed in the fan box 34.

Moreover, a deodorizing unit, which realizes complete combustion of odorous particles contained in moist air drawn into the suction pipe 30 and oxidizes the odorous particles, is provided on the suction pipe 30 between the air circulation device 20 and the fan box 34.

In detail, as shown in FIGS. 2 and 3, the deodorizing unit includes a cylindrical deodorization housing 40, which has a structure such that an inlet, connected to the suction pipe 30, and an outlet thereof are disposed at positions maximally spaced apart from each other. The deodorizing unit further includes a catalytic heater 42, which is installed in the deodorization housing 40 and reheats drawn moist air containing odorous particles therein, and a deodorizing plate 44, which is provided on a circumferential outer surface of the catalytic heater 42 to oxidize odorous particles. The deodorizing plate 44 has a screw shape so as to increase both the time taken until moist air drawn into the deodorization housing 40 is discharged through the outlet thereof, and the contact surface area between the deodorizing plate 44 and the moist air.

Furthermore, a thermal insulation member 48 is additionally provided on the outer surface of the deodorization housing 40 so as to prevent the loss of heat generated by the catalytic heater 42 and prevent peripheral elements from being damaged.

It is preferable that the deodorization housing 40 be made of stainless steel to ensure superior corrosion resistance against drawn moist air and salt and to increase the thermal conductivity with respect to the catalytic heater 42.

Furthermore, it is preferable that the deodorizing plate 44 be made of SUS (special use stainless steel), and that zeolite, alumina carrier and noble metal catalyst, or silica catalyst be applied to the outer surface thereof.

As well, preferably, the catalytic heater 42 has a heating temperature range of 350°C or more.

In this embodiment, a deodorization temperature sensor 46, which detects the temperature of the interior of the deodorization housing 40 that is heated by the catalytic heater 42, is provided on the outer surface of the deodorization housing 40. Detection values sensed by the temperature sensor 46 are used as comparative values such that a control unit, which is not shown in the drawings, automatically turns on/off the catalytic heater 42 while the apparatus is in operation.

Meanwhile, a geared motor 50, which is coupled to a reduction gear, is vertically installed at a lower position in the main body 10. A rotating shaft 52 is disposed adjacent to the geared motor 50 so as to be rotatable using bearings. Two belt pulleys 54, which are coupled to each other through a belt to transmit rotating force therebetween, are respectively provided on an output end of the geared motor 50 and a lower end of the rotating shaft 52.

Furthermore, an agitation means is provided on the bottom of the food waste receiving tub 16. The agitation means agitates food waste contained in the food waste receiving tub 16, thus reducing the heating temperature difference between the upper portion and the lower portion of the food waste, and helping eliminate moisture. The agitation means includes an agitating shaft 56, which is selectively and removably coupled to the rotating shaft 52 by a key so as to be rotatable, and an agitating blade 58, which is provided on an end of the agitating shaft 56.

In the drawings, the reference numeral 19 denotes a grill net, which serves to prevent food waste from leaking outside the food waste receiving tub 16 after the food waste has been completely agitated and dried. The grill net 19 has a removable structure, so that it allows easy clean-up.

The operation of the food waste drying apparatus of the present invention having the above-mentioned construction will be explained herein below.

After an appropriate amount of food waste has been input into the food waste receiving tub 16, the upper lid 12 is closed, and power is applied to the apparatus by manipulating the control panel 12a. Then, power is applied both to electric units (the circulation fan, the suction fan, the rotating shaft and the agitating shaft and agitating blade), which are operated by the motors, and to the heaters (the main heater and the catalytic heater), which are operated in a preprogrammed operation order by the control unit (not shown).

To realize the rotation of the agitating blade 58, the rotation of the geared motor 50, which serves as a power source, is transmitted to the rotating shaft 52 through the belt pulleys 54, which are provided on the output end of the motor and the end of the rotating shaft 52 and are coupled to each other. The rotating force of the rotating shaft 52 is transmitted to the agitating blade 58 through the agitating shaft 56, which is coupled to the rotating shaft 52 by the key.

While these elements are operated, the food waste contained in the food waste receiving tub 16 is heated by the main heater 24 and the circulation fan 22. At this time, moist steam generated in the receiving tub 16 is drawn into the air circulation device by the rotation of the circulation fan 22. Some air is again supplied into the food waste receiving tub 16 by the circulation fan 22 via the main heater 24, and some air is discharged outside through the deodorizing unit. As such, the food waste drying apparatus of the present invention is constructed such that thermal efficiency is markedly enhanced.

Meanwhile, moist air generated from the food waste is rapidly discharged outside by the suction fan 32, which is provided at a rear position in the main body 10. Here, in a drying process, odorous particles as well as moist air are generated from the food waste.

Such odorous particles are completely oxidized both by moving past the deodorizing plate 44, which has a screw structure, so that odorous particles remain in the deodorization housing 40 as long as possible, and has a plate shape to increase the contact surface area between it and the odorous particles, and to which zeolite, alumina carrier and noble metal catalyst, or silica catalyst is applied, and by moving past the catalytic heater 42, which is heated to 350°C or more. Moist air is also discharged outside after being changed into dry air by passing through the deodorization housing 40.

The deodorization housing 40 and the deodorizing plate 44 are made of SUS (special use stainless steel) having superior corrosion resistance, thus being prevented from corroding due to salt condensed in the process of heating and drying moist air.

The operation time required for drying food waste contained in the food waste receiving tub 16 varies depending on the amount of food waste. Furthermore, after the time required for drying food waste is arbitrarily determined by the moist air sensor 28a, mounted to the induction pipe 28 of the air circulation device 20, depending on the temperature difference between moist steam and dry air, the apparatus is automatically operated.

After the process of drying the food waste is completed, the upper lid 12 is opened. Thereafter, the food waste receiving tub 16 can be easily removed, because the rotating shaft 52 and the agitating shaft 56 are separably coupled to each other by the coupling method using the key and key groove.

The present invention having the above-mentioned construction and operation has the following advantages.

First, the present invention uses a double heating method both using a main heater, which is disposed at an upper position around a food waste receiving tub, and using a catalytic heater, which is disposed at a lower position thereof. In addition, an agitating blade agitates food waste contained in the food waste receiving tub during the drying process. Therefore, the drying efficiency thereof is maximized, so that the electric heating cost is markedly reduced compared to the conventional art.

Second, because a deodorizing unit of the present invention dries and eliminates moist air and odorous particles, which are generated during the drying process, in a short time, environmental pollution is prevented, thus minimizing damage to neighbors.

Although the preferred embodiment of the present invention has been disclosed for illustrative purposes, those skilled in the art will appreciate that various modifications, additions and substitutions are possible, without departing from the scope and spirit of the invention as disclosed in the accompanying claims.

## Claims

1. A food waste drying apparatus having a main body; a food waste receiving tub seated on a support provided in the main body; a heater to heat and dry food waste contained in the food waste receiving tub; and a suction fan and a suction pipe to discharge heated moist air therethrough to an outside, the food waste drying apparatus comprising:
a rotating shaft provided on the support and rotates using a driving force transmitted from a drive motor;
agitation means provided on the food waste receiving tub and removably coupled to the rotating shaft, so that the agitation means agitates the food waste, thus reducing a temperature difference in the food waste, thereby increasing a drying efficiency; and
a deodorizing unit provided in a path of the suction pipe to burn the moist air, which contains odorous particles therein and is discharged through the suction pipe.

2. The food waste drying apparatus as set forth in claim 1, wherein the agitation means comprises:
an agitating shaft keyed to the rotating shaft and provided so as to be rotatable; and
an agitating blade provided on an end of the agitating shaft.

3. The food waste drying apparatus as set forth in claim 1, wherein the deodorizing unit comprises:
a cylindrical deodorization housing, with an inlet and an outlet formed in the cylindrical deodorization housing at positions facing each other;
a catalytic heater provided in the cylindrical deodorization housing to reheat the drawn moist air including the odorous particles; and
a deodorizing plate provided on the catalytic heater to oxidize the odorous particles contained in the moist air and having a zigzag shape to increase both a time, taken until the moist air drawn into the deodorization housing is discharged through the outlet, and a contact surface area thereof.

4. The food waste drying apparatus as set forth in claim 3, wherein the deodorization housing is made of SUS (special use stainless steel) to increase corrosion resistance against the drawn moist air and salt and to increase thermal conductivity with respect to the catalytic heater.

5. The food waste drying apparatus as set forth in claim 3, wherein the deodorizing plate is made of SUS (special use stainless steel), and zeolite, alumina carrier and noble metal catalyst, or silica catalyst is applied to an outer surface of the deodorizing plate.
